# EUROPÄISCHE PATENTANMELDUNG

(11) **EP 4 693 861 A1**
(43) Veröffentlichungstag der Anmeldung: **11.02.2026**
(21) Anmeldenummer: 25193387.5
(22) Anmeldetag: 01.08.2025
(51) Int. Cl.: H02M 1/36, H02M 1/32

(54) **VERSORGUNGSVORRICHTUNG ZUR ENERGIEVERSORGUNG EINES LASTMODULS**

(30) Priorität: 08.08.2024 DE 102024122647
(71) Anmelder: Dräger Safety AG & Co. KGaA, 23560 Lübeck (DE)
(72) Erfinder: Schulze, Martin, 23558 Lübeck (DE)

(57) **Zusammenfassung**

Ein Lastmodul (200) zur Versorgung einer Last weist einen Eingangsanschluss (210) zur Ankopplung an eine Energieversorgung und einen Ausgangsanschluss (220) zur Ankopplung an die Last auf. Eine Schalteinrichtung (230) dient zur variablen elektrischen Kopplung des Eingangsanschlusses (210) mit dem Ausgangsanschluss (220). Eine Kontrolleinrichtung (240), die mit Schalteinrichtung (230) gekoppelt ist, ist ausgebildet, um nach einer Ankopplung des Lastmoduls (200) an eine Energieversorgung über die Schalteinrichtung (230) eine elektrische Kopplung des Eingangsanschlusses (210) mit dem Ausgangsanschluss (220) zu kontrollieren.

## Beschreibung

Die vorliegende Erfindung betrifft die Versorgung von Lasten mit Strom, beispielsweise von Lasten, die sich in explosionsgefährdeten Bereichen befinden.

Beispielsweise werden Transmitter in Sensorköpfen oder Transmitter zur Kommunikation mit Sensorköpfen zum Messen von brennbaren und explosionsfähigen Gasen in weiten Teilen der Industrie eingesetzt, um frühzeitig brennbare, toxische und explosionsfähige Gase zu detektieren. Ziel dieser Detektion ist es Bereiche rechtzeitig zu evakuieren oder entsprechende Gegenmaßnahmen einzuleiten. Hierzu zählen bspw. Akustische und optische Alarmierung sowie die Einleitung von Zwangsventilation oder Abschaltung bestimmter Anlagen. Einige dieser Transmitter werden auch in der Prozessmesstechnik eingesetzt und sind Teil der Regelungskette in der petrochemischen Industrie. Ein gesamtes System zum Messen von Gasen besteht zumeist aus einem Controller, einem Barrieremodul und dem eigentlichen Transmitter. Der Transmitter überträgt den Messwert häufig über Stromschleife (Schnittstelle 4-20mA) zum Controller, indem er einen Strom abhängig vom eigentlichen Messwert in die Leitung einprägt. Bei solchen 2-Draht Transmittern versorgt sich das Gerät häufig aus dem eingeprägten Strom der Stromschleife.

Der Transmitter versorgt einen abgesetzten Messkopf mit Strom und kommuniziert mit diesem. Aus Sicht des Transmitters ist der abgesetzte Messkopf dann eine Last, die er mit Strom versorgt. Ein solcher Transmitter dient also auch Versorgungsvorrichtung zur Energieversorgung der durch den abgesetzten Sensorkopf erzeugten Last. Im abgesetzten Sensorkopf selbst befindet sich wiederum ein Transmitter zur Kommunikation mit dem Transmitter der Versorgungsvorrichtung. Da die Kommunikation und die Energieversorgung über dieselben Kabel erfolgt, befindet er sich zwischen der Versorgungsvorrichtung zur Energieversorgung und dem Sensor selbst, der ebenfalls über die Kabel mit Strom versorgt wird. Dieser Transmitter kann also auch als ein Lastmodul zur Versorgung einer Last betrachtet werden, insbesondere zur Versorgung des Sensors mit Strom.

Diese Transmitter werden unter sehr unterschiedlichen Umgebungsbedingungen eingesetzt. Sie können auch in den Umgebungen mit den potenziell explosionsfähigen Gasen oder Stäuben positioniert werden, welche besondere Ansprüche an verwendeten Komponenten stellen. Zum Explosionsschutz müssen zwei Gründe für eine potenzielle Zündung ausgeschlossen werden. Die Funkenentzündung, zumeist durch die Begrenzung der im Fehlerfall freiwerdenden Energie und eine Temperaturbegrenzung, die die Selbstentzündung ausschließt.

Ein ähnlicher Anwendungsfall der Stromversorgung betrifft Pumpen, die in der stationären Gasmesstechnik dazu eingesetzt werden, eine kleine Menge Gas aus einer Anlage bzw. einem Rohr zu ziehen und diesen zu einem Sensor zu fördern. Oft sind Pumpenanwendungen dadurch gekennzeichnet, dass die Probenahme in einem sehr unzugänglichen Raum vorgenommen wird und das Gas durch Schläuche oder Rohre über einen längeren Weg zur eigentlichen Messtelle transportiert wird. Da solche Pumpen häufig in Bereichen mit explosiver Atmosphäre eingesetzt werden und zudem Gasgemische fördern, die potenziell zündfähig sind, bestehen bei der Konstruktion solcher Pumpen erhöhte Anforderungen, damit diese nicht selbst zur Zündquelle werden.

Auch bei der Anwendung als Stromversorgung für Pumpen in derartigen Atmosphären besteht also eine Notwendigkeit, die Energieversorgung des Pumpenmoduls derart zu gestalten, dass diese nicht als Zündquelle dienen kann.

DE 11 2011 101 763 T5 offenbart ein System zur Stromversorgung einer Abzweigleitung in einem Feldbussystem mit einer Strommesseinrichtung, einer Steuereinrichtung und einer Verarbeitungseinrichtung, die den Strom basierend auf der Messung steuert.

DE 20 2019 000 194 U1 offenbart eine Schaltung zur Reduzierung von Einschaltströmen durch zeitlich versetztes Einschalten von Verbrauchern nach dem Anlegen einer Versorgungsspannung.

Zusammengefasst besteht ein Bedarf daran, die Energieversorgung von Lasten oder Geräten derart zu ermöglichen, dass die Energieversorgung auch im Fehlerfall keine Zündquelle darstellt.

Dieser Bedarf wird durch ein in dem unabhängigen Anspruch definiertes Lastmodul und durch das dazu korrespondierende Verfahren gedeckt.

Einige der Ausführungsbeispiele basieren dabei auf der Erkenntnis, dass es möglich ist, mittels geeigneter Ausführung der als Energiequelle dienenden Versorgungsvorrichtung und des Lastmoduls zu verhindern, dass selbst bei einer Fehlfunktion so große Energiemengen freiwerden können, dass dadurch eine Zündquelle entstünde.

Offenbart wird außerdem eine Versorgungsvorrichtung zur Energieversorgung eines Lastmoduls umfasst einen Eingangsanschluss zur Ankopplung an eine Energieversorgung und einen Ausgangsanschluss zur Energieversorgung des Lastmoduls. Eine Messeinrichtung dient der Messung einer Stromaufnahme über den Eingangsanschluss und eine Steuereinrichtung zur Steuerung eines Stroms über den Ausgangsanschluss. Eine Kontrolleinrichtung ist mit der Messeinrichtung und der Steuereinrichtung gekoppelt und ausgebildet, um den Strom über den Ausgangsanschluss basierend auf der Stromaufnahme über den Eingangsanschluss zu kontrollieren. Wird der Strom kontrolliert, kann beispielsweise die Energiemenge so begrenzt werden, dass diese selbst im Fehlerfall wie bei einem Kurzschluss der mittels der Versorgungsvorrichtung gespeisten Leitungen nicht ausreicht, um ein Medium im Umfeld der Leitungen oder in der Umgebung eines Gesamtsystems zu zünden.

Gemäß einigen Ausführungsbeispielen ist die Kontrolleinrichtung zu diesem Zweck ausgebildet, um den Strom über den Ausgangsanschluss zu begrenzen.

Gemäß einigen Ausführungsbeispielen ist die Kontrolleinrichtung ausgebildet, um bei Überschreiten eines Schwellenwertes der Stromaufnahme über den Eingangsanschluss den Strom über den Ausgangsanschluss zu begrenzen.

Gemäß einigen Ausführungsbeispielen ist die Kontrolleinrichtung ausgebildet ist, um über die Steuereinrichtung eine elektrische Kopplung des Eingangsanschlusses mit dem Ausgangsanschluss zu kontrollieren. Die Kontrolle der Kopplung kann beispielsweise dazu verwendet werden, den Strom nicht vollständig abzuschalten, sondern diesen zwischen Eingangsanschluss und Ausgangsanschluss den Erfordernissen entsprechend zu begrenzen, sodass gegebenenfalls ein Betrieb fortgesetzt werden kann, auch wenn sich der Stromverbrauch ohne Kontrolle der Kopplung außerhalb eines gewünschten Bereichs bewegen würde.

Gemäß einigen Ausführungsbeispielen ist die Kontrolleinrichtung ausgebildet, um über die Steuereinrichtung eine elektrische Kopplung des Eingangsanschlusses mit dem Ausgangsanschluss zu unterbrechen, um so beispielsweise maximale Sicherheit zu gewährleisten.

Zusätzlich kann die Versorgungsvorrichtung gemäß einigen Beispielen druckfest gekapselt sein, sodass diese selbst in Fehlerfall zusätzlich geschützt ist.

Erfindungsgemäß umfasst das Lastmoduls zur Versorgung einer Last einen Eingangsanschluss zur Ankopplung an eine Energieversorgung und einen Ausgangsanschluss zur Ankopplung an die Last. Eine Schalteinrichtung dient zur variablen elektrischen Kopplung des Eingangsanschlusses mit dem Ausgangsanschluss. Eine Kontrolleinrichtung ist mit der Schalteinrichtung gekoppelt und ausgebildet, um nach einer Ankopplung des Lastmoduls an eine Energieversorgung über die Schalteinrichtung eine elektrische Kopplung des Eingangsanschlusses mit dem Ausgangsanschluss zu kontrollieren. Die Möglichkeit der Kontrolle kann dazu genutzt werden, zu verhindern, dass Ströme fließen, die so beschaffen sind, dass sie eine Zündgefahr darstellen.

Erfindungsgemäß ist die Kontrolleinrichtung ausgebildet, um einen Stromfluss mittels eines geschalteten Transistors nur in einer Richtung zu ermöglichen. Dies kann beispielsweise verhindern, dass im Fall einer Fehlfunktion Umladeströme fließen, die so groß sein könnten, dass sie, beispielsweise an der Stelle eines Kurzschlusses in einer Zuleitung, zu einer Zündung eines Gasgemisches führen können.

Gemäß einigen Ausführungsbeispielen ist die Kontrolleinrichtung ausgebildet, um nach der erfolgten Ankopplung an die Energieversorgung über die Schalteinrichtung eine elektrische Kopplung des Eingangsanschlusses mit dem Ausgangsanschluss zu verzögern. Dies kann verhindern, dass Anlaufströme von Maschinen oder Ladeströme von Kapazitäten dazu führen, dass eine kritische Stromstärke nach der Ankopplung an eine Energieversorgung überschritten wird.

Gemäß einigen Ausführungsbeispielen ist die Kontrolleinrichtung ausgebildet, um die verzögerte elektrische Kopplung des Eingangsanschlusses mit dem Ausgangsanschluss basierend auf einem Ladezustand eines Kondensators durchzuführen. Die Verwendung eines geeignet großen Kondensators kann so beispielsweise dazu dienen, zu bestimmen, wann die Kapazitäten einer Last aufgeladen sind, sodass bei einer niederohmigen Verbindung zwischen dem Eingangsanschluss und dem Ausgangsanschluss danach keine hohen Ladeströme mehr auftreten.

Optional kann daher der Kondensator nach Ankopplung an die Energieversorgung über einen begrenzten Strom geladen wird.

Gemäß einigen Ausführungsbeispielen kann dies robust und effizient erreicht werden, indem die Schalteinrichtung mit einem elektrischen Widerstand überbrückt ist, um den Kondensator zu laden.

Gemäß einigen Ausführungsbeispielen ist die Kontrolleinrichtung ausgebildet, um die Schalteinrichtung hochohmig zu schalten, wenn die Ankopplung an die Energieversorgung erfolgt, und um die Schalteinrichtung niederohmig zu schalten, wenn die Kondensatorspannung einen vordefinierten Schwellwert überschreitet. Wird nach dem initialen Aufladen des Kondensators die Schalteinrichtung niederohmig, kann eine Leitungslänge zur Anbindung des Lastmoduls verlängert werden als in dem Fall, in dem die zumindest zu Beginn aus Sicherheitsgründen die Hochohmigkeit bestehen bleibt.

Einige Beispiele von Vorrichtungen und/oder Verfahren werden nachfolgend bezugnehmend auf die beiliegenden Figuren näher erläutert. Es zeigen:
Fig. 1 ein Blockdiagramm eines Beispiels einer Versorgungsvorrichtung;
Fig. 2 ein Blockdiagramm der Versorgungsvorrichtung von Fig. 1 und eines Ausführungsbeispiels eines Lastmoduls;
Fig. 3 eine weiteres Ausführungsbeispiel eines Lastmoduls mit der Versorgungsvorrichtung der Fig.1;
Fig. 4 ein Flussdiagramm eines Ausführungsbeispiels eines Verfahrens zur Energieversorgung eines Lastmoduls; und
Fig. 5 ein Flussdiagramm eines Ausführungsbeispiels eines Verfahrens zur Ansteuerung einer Last.

Verschiedene Beispiele werden nun ausführlicher Bezug nehmend auf die beiliegenden Figuren beschrieben. In den Figuren können die Stärken von Linien, Schichten und/oder Bereichen zur Verdeutlichung übertrieben sein.

Weitere Beispiele können Modifikationen, Entsprechungen und Alternativen abdecken, die in den Rahmen der Offenbarung fallen. Gleiche oder ähnliche Bezugszeichen beziehen sich in der gesamten Beschreibung der Figuren auf gleiche oder ähnliche Elemente, die bei einem Vergleich miteinander identisch oder in modifizierter Form implementiert sein können, während sie die gleiche oder eine ähnliche Funktion bereitstellen.

Es versteht sich, dass, wenn ein Element als mit einem anderen Element "verbunden" oder "gekoppelt" bezeichnet wird, die Elemente direkt, oder über ein oder mehrere Zwischenelemente, verbunden oder gekoppelt sein können. Wenn zwei Elemente A und B unter Verwendung eines "oder" kombiniert werden, ist dies so zu verstehen, dass alle möglichen Kombinationen offenbart sind, d. h. nur A, nur B sowie A und B, sofern nicht explizit oder implizit anders definiert. Eine alternative Formulierung für die gleichen Kombinationen ist "zumindest eines von A und B" oder "A und/oder B". Das Gleiche gilt, mutatis mutandis, für Kombinationen von mehr als zwei Elementen.

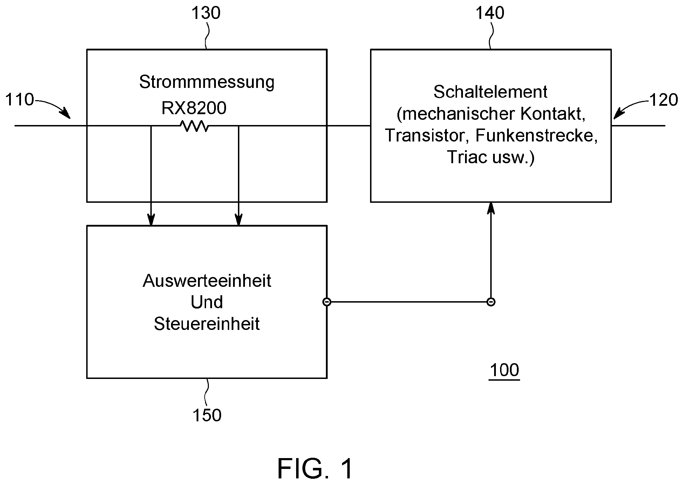

Fig. 1 zeigt ein Blockdiagramm eines Beispiels einer Versorgungsvorrichtung 100 zur Energieversorgung eines Lastmoduls.

Diese weist einen Eingangsanschluss 110 zur Ankopplung an eine Energieversorgung sowie einen Ausgangsanschluss 120 zur Energieversorgung des Lastmoduls auf. Eingangsanschlüsse und Ausgangsanschlüsse für Strom können auf verschiedene Weise gestaltet werden, abhängig von den spezifischen Anforderungen der Anwendung. Beispielsweise können diese in Form eines Standardsteckers für Geräteanschlüsse (z.B. Kaltgeräteanschluss IEC 60320) oder als Schuko-Stecker ausgebildet sein. Weitere Möglichkeiten sind Netzteil-Stecker, also beispielsweise runde Stecker für verschiedene Spannungen. Möglich sind aber auch Klinkenstecker sowie Schraubklemmen oder Federkraftklemmen. Bananenstecker und modulare Steckverbinder, die in vielen Konfigurationen zusammengesteckt werden können, sind eine weitere Möglichkeit, diese Anschlüsse zu implementieren.

Die Versorgungsvorrichtung 100 umfasst ferner eine Messeinrichtung 130 zur Messung einer Stromaufnahme über den Eingangsanschluss 110 sowie eine Steuereinrichtung 140 zur Steuerung eines Stroms über den Ausgangsanschluss 120. Der Strom kann je nach Anwendung auf unterschiedliche Weise gemessen werden. Beispiele dafür wären die Verwendung eines Shunt-Widerstands, eines Stromspiegels, von Hall-Effekt-Sensoren oder eines integrierten Strommess-ICs.

Zur Steuerung, also beispielsweise zur Begrenzung oder Abschaltung von Strom können in der Steuereinrichtung 140 verschiedene Methoden, um den Stromfluss in einem Stromkreis zu kontrollieren, bzw. zu steuern oder ihn an- und abzuschalten können, implementiert werden. Beispielsweise können Relais verwendet werden. Elektromechanische Relais nutzen eine elektromagnetische Schaltung, um größere Ströme zu steuern, während Solid-State-Relais (SSR) ohne bewegliche Teile arbeiten und somit schneller und langlebiger sind. Transistoren, wie Bipolartransistoren (BJT) und Feldeffekttransistoren (FET), sind ebenfalls weit verbreitete elektronische Schalter. Sie können durch kleine Steuerströme große Lastströme schalten, wobei MOSFETs oft in digitalen Schaltungen verwendet werden. Für höhere Spannungen und Ströme sind IGBT (Insulated Gate Bipolar Transistor) und GTO (Gate Turn-Off Thyristor) geeignet. Thyristoren, wie der Silicon Controlled Rectifier (SCR) und der Triac, bieten robuste Lösungen für das Schalten von Wechselstrom, insbesondere in Dimmern und anderen Steuerungen.

Eine Kontrolleinrichtung 150 ist mit der Messeinrichtung 130 und der Steuereinrichtung 140 gekoppelt und ausgebildet ist, um den Strom über den Ausgangsanschluss 120 basierend auf der Stromaufnahme über den Eingangsanschluss 110 zu kontrollieren. Die Kontrolleinrichtung 150 empfängt Messwerte, die auf den Stromfluss schließen lassen und steuert die Steuereinrichtung 140. Die Kontrolleinrichtung 150 implementiert eine Logik zur Kontrolle des Stromflusses. Die Logik könnte mit verschiedensten Technologien implementiert werden. Eine der grundlegenden Technologien hierfür wären diskrete Logikgatter, die aus Transistoren bestehen und einfache logische Funktionen wie AND, OR und NOT realisieren. Eine weitere Form der Logikimplementierung sind integrierte Schaltkreise (ICs), wie sie in Form von TTL (Transistor-Transistor-Logik) und CMOS (Complementary Metal-Oxide-Semiconductor) existieren. Für anpassbare Logikschaltungen können programmierbare Logikbausteine wie FPGAs (Field-Programmable Gate Arrays) verwendet werden. Eine weitere Möglichkeit zur Implementierung dieser Logik ist die Verwendung von Mikrocontrollern und Mikroprozessoren. Auch spezialisierte ICs, wie Application-Specific Integrated Circuits (ASICs), die speziell für eine bestimmte Anwendung oder ein bestimmtes Set von Aufgaben entwickelt werden, können dafür verwendet werden.

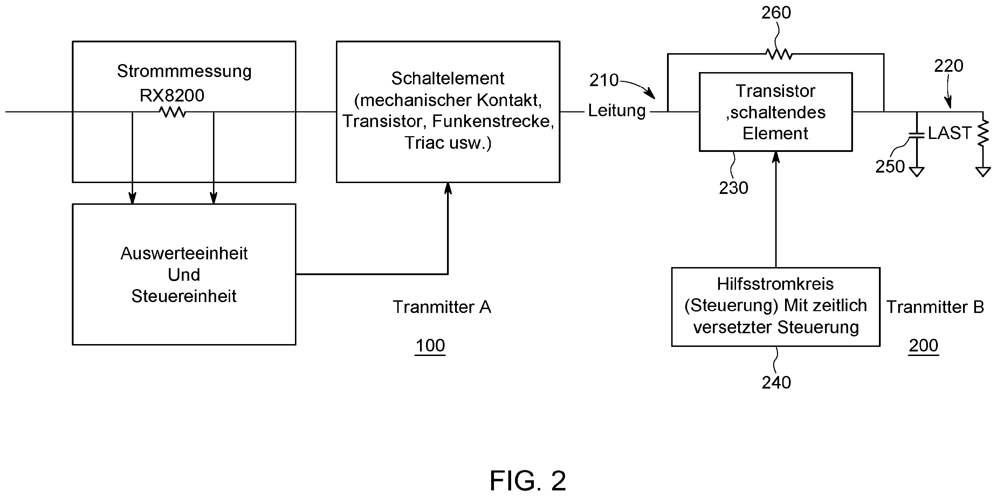

Fig. 2 zeigt ein Blockdiagramm der Versorgungsvorrichtung 100 von Fig. 1 und eines Ausführungsbeispiels eines Lastmoduls 200, das mit einer Versorgungsvorrichtung 100 verbunden ist.

Das Lastmodul 200 dient der Versorgung einer Last mit Energie und in manchen Beispielen auch der Kommunikation mit der Versorgungsvorrichtung 100 über die Leitung oder die Leitungen, die die Energie transportieren. In dieser Funktion kann das Lastmodul 200 auch als Transmitter verstanden werden.

Das Lastmodul 200 weist einen Eingangsanschluss 210 zur Ankopplung an eine Energieversorgung und einen Ausgangsanschluss 220 zur Ankopplung an die Last auf. Eine Schalteinrichtung 230 dient der variablen elektrischen Kopplung des Eingangsanschlusses 210 mit dem Ausgangsanschluss 220. Eine Kontrolleinrichtung 240 ist mit der Schalteinrichtung 230 gekoppelt und ausgebildet, um nach einer Ankopplung des Lastmoduls 200 an eine Energieversorgung (beispielsweise die Versorgungsvorrichtung 100) über die Schalteinrichtung 230 eine elektrische Kopplung des Eingangsanschlusses 210 mit dem Ausgangsanschluss 220 zu kontrollieren.

Als Technologien zur Implementierung der Schalteinrichtung 230 können beispielsweise diejenigen verwendet werden, die anhand der Fig. 1 für die Steuereinrichtung 140 diskutiert wurden. Als Technologien zur Implementierung der Kontrolleinrichtung 240 können beispielsweise diejenigen verwendet werden, die anhand der Fig. 1 für die Kontrolleinrichtung 150 diskutiert wurden.

Die Ankopplung an die Energieversorgung kann auf beliebige Art und Weise detektiert werden, beispielsweise mit der Messung einer Spannung oder eines Stroms am Eingangsanschluss 210. Die Ankopplung kann auch implizit bestimmt werden, beispielsweise dann angenommen werden, wenn eine Logik in der Kontrolleinrichtung 240 zum ersten Mal mit Energie versorgt wird und beginnt, zu arbeiten. Prinzipiell möglich ist auch eine mechanische Detektion, die beispielsweise durch ein Anstecken eines Steckers ausgelöst wird.

In dem in Fig. 2 gezeigten Ausführungsbeispiel ist die Kontrolleinrichtung 240 ausgebildet, um nach der erfolgten Ankopplung an die Energieversorgung über die Schalteinrichtung 230 eine elektrische Kopplung des Eingangsanschlusses 210 mit dem Ausgangsanschluss 220 zu verzögern. So kann sichergestellt werden, dass unmittelbar nach Beginn der Energieversorgung Anlaufströme oder Ladeströme von Kapazitäten nicht dazu führen, dass für den Explosionsschutz nicht mehr akzeptable Energien über die Leitungen übertragen werde. Danach können höhere Ströme als bei konventionellen Lösungen übertragen werden, die die Strombegrenzung während der gesamten Betriebsdauer aufrechterhalten, beispielsweise mittels eines Widerstands. Alternativ kann der niedrigere Leistungsverlust auch dadurch ausgenutzt werden, dass die Länge der Zuleitung zu dem Lastmodul 200 erheblich größer sein kann als bei herkömmlichen Implementierungen.

In dem in Fig. 2 gezeigten Fall wird die Kopplung des Eingangsanschlusses 210 mit dem Ausgangsanschluss 220 basierend auf einem Ladezustand des Kondensators 250 durchgeführt. Über den Widerstand 260, der die Schalteinrichtung 230 überbrückt, wird der Kondensator 250 nach Ankopplung an die Energieversorgung zunächst über einen begrenzten Strom geladen. Während dieser Zeit trennt die Schalteinrichtung 230 den Eingangsanschluss 210 von dem Ausgangsanschluss 220. Erreicht der Kondensator 250 einen festgelegten Ladezustand (beispielsweise gemessen durch den Spannungsabfall über den Kondensator), verbindet die Schalteinrichtung 230 den Eingangsanschluss 210 mit dem Ausgangsanschluss 220. Alternativ kann die Verzögerung auch anders implementiert werden, beispielsweise, indem eine bestimmte Zeit gewartet wird.

Allgemeiner gefasst kann das Konzept des Lastmoduls 200 der Fig. 2 dergestalt beschreiben werden, dass das die Kontrolleinrichtung ausgebildet, um die Schalteinrichtung 230 hochohmig zu schalten, wenn die Ankopplung an die Energieversorgung erfolgt, und um die Schalteinrichtung 230 niederohmig zu schalten, wenn die Kondensatorspannung einen vordefinierten Schwellwert überschreitet.

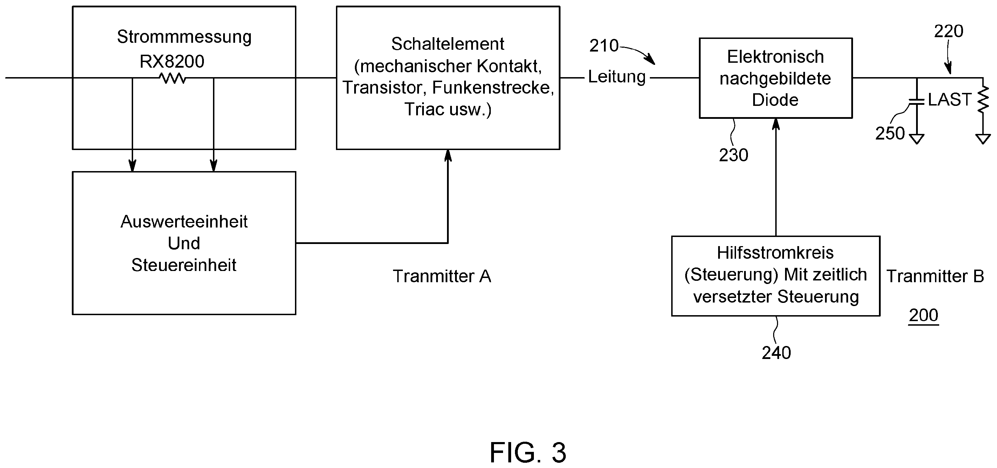

Fig. 3 zeigt eine weiteres Ausführungsbeispiel eines Lastmoduls 200, das in wesentlichen Teilen dem in Fig. 2 gezeigten Ausführungsbeispiel entspricht. Daher sind gleiche Komponenten auch mit gleichen Bezugszeichen versehen. Anders als im in Fig. 2 beschriebenen Fall ist die Kontrolleinrichtung 240 zusätzlich oder alternativ ausgebildet, um einen Stromfluss nur in einer Richtung zu ermöglichen. Beispielsweise kann so verhindert werden, dass im Falle eines Kurzschlusses in der Leitung zum Eingangsanschluss 210 Umladeströme in dem Lastmodul 200 oder in der an dieses angeschlossenen Last zu einer Funkenbildung führen können. In dem Fall wird beispielsweise ein Stromfluss in den Eingangsanschluss 210 hinein erlaubt, im Fall eines möglichen Stromflusses aus dem Eingangsanschluss 210 heraus jedoch der Eingangsanschluss 210 vom Ausgangsanschluss 220 getrennt. Die Messung der Stromflussrichtung kann dabei auf beliebige bekannte Art und Weise erfolgen.

Wie eingangs dargelegt, können mögliche Lasten, die von dem Lastmodul versorgt werden bzw. an dieses angeschlossen sind, Pumpenmodule oder Messköpfe für Gase sein. Darauf ist dieses Konzept jedoch nicht beschränkt. Vielmehr können beliebige Lasten mit den hierin beschriebenen Lastmodulen gespeist bzw. betrieben werden.

In anderen Worten zeigen die Fig. 2 und die Fig. 3 jeweils zwei räumlich voneinander separierte Teilen zur Energieversorgung.

Die Versorgungsvorrichtung 100 ist ein Transmitter A, der auch eine nicht dargestellte Verbindung zu einem Controller des Systems halten kann. Das Lastmodul 200 kann ebenfalls als ein Transmitter B / Messkopf betrachtet werden, der abgesetzt von Transmitter A über eine Verdrahtung an Transmitter B angeschlossen ist.

Auf Seite A wird durch eine Schaltung eine Strommessung durchgeführt. Die Steuerung kann durch einen Hilfsstromkreis erfolgen, der die Auswertung des Strommessung übernimmt. In der Auswerteeinheit ist ein Schwellwertschalter integriert. Bei Überschreiten eines der Anwendung entsprechenden Schwellwertes schaltet die Steuereinheit das nachfolgend an die Strommessung angeordnete Schaltelement ab. Das Abschalten des Schaltelements führt wirkt dem Anstieg des Stroms entgegen. Dabei ist nicht von Belang, ob das Schalten in Form eines Öffnerkontaktes ausgeprägt ist oder in Form einer linear steuerbaren Strombegrenzung existiert.

Im in Fig. 3 gezeigten Fall ist im Transmitter B ist eine elektronische Schaltung integriert, die den Strom der versorgenden Leitung nur in eine Richtung durchlässt. Sie könnte optional auch durch einen Widerstand überbrückt sein, der einen gewissen Grundstrom zulässt. Ein Hilfsstromkreis könnte in diesem Fall die Funktion der Steuerung übernehmen und dafür sorgen, dass sich die Schalteinrichtung 230, z.B. ein in Ihre verwendeter Transistor wie eine Diode verhält, also den Strom nur in einer Richtung durchlässt, allerdings mit sehr viel geringerem Spannungsabfall, wodurch die hohe Energieeffizient erreicht wird.

Im in Fig. 2 gezeigten Fall ist der Zustand der Logik der Kontrolleinrichtung 240 so gewählt, dass das schaltende Element keinen Strom durchlässt. Schaltet man nun die Betriebsspannung ein, so wird der Strom zunächst nur vom überbrückenden Widerstand bestimmt. Das strombegrenzende Bauteil kann in der konkreten Implementierung ein Widerstand, eine Induktivität oder ein zum Teil aufgesteuerter Transistor sein, sowie eine beliebige Kombination aus diesen Bauteilen. Dieser Strom ist in der Regel eine Größenordnung kleiner als der im Transmitter A eingestellte Strom der Strombegrenzung.

Der begrenzte Strom kann nun die die Ladungsspeicher der angeschlossenen Last auf, bis der Schaltpunkt erreicht wird. Dieser schaltet zeitverzögert das schaltende Element bzw. die Schalteinrichtung 230 frei. Damit ist dann eine niederohmige Verbindung und somit eine annähernd verlustfreie Übertragung zwischen Transmitter A und Transmitter B möglich.

Alternativ kann das strombegrenzende Bauteil und das schaltende Element ein und derselbe Transistor sein, der zur Strombegrenzung zuerst teilweise aufgesteuert wird und danach voll durchgesteuert wird. Außerdem ist es möglich, dass dieser Übergang nicht in Stufen, sondern kontinuierlich durchgeführt wird.

Durch diese Ausprägung der Erfindung reduziert sich der Umladestrom im Vergleich zu herkömmlichen Implementierungen um Größenordnungen. Dadurch können beispielsweise Schmelzsicherungen vor unbeabsichtigtem Auslösen geschützt werden. Dadurch wird nicht nur die Funkenzündung unterbunden, sondern zusätzlich noch ein Soft-Start realisiert. Dieser erlaubt den Anschluss hoher kapazitiver Lasten, die andernfalls dazu führen könnten, dass die elektronische Sicherung unabsichtlich ausgelöst wird.

Durch dieses Konzept ist es möglich, zwei Transmitter im explosionsgefährdeten Bereich zu verbinden, bei gleichzeitig hoher Energieeffizienz und Vermeidung von Funkenbildung. Sämtliche Kurzschlüsse im Kabel führen zu keiner Funkenzündung. Die mögliche Länge der Verbindung wird ebenfalls erheblich gesteigert. Mit herkömmlichen Ansätzen zum Schutz vor Funkenbildung reduziert sich in ähnlichen Anwendungen die Kabellänge zwischen Transmitter A und Transmitter B bedingt durch die reale Induktivität des Kabels unter gleichen Bedingungen deutlich.

Auch kann dadurch die Anforderung an die zu verwenden Kabel und Planung von Anlagen deutlich reduziert werden, wodurch geringere Systemkosten erzielt werden können. Kabel könnten so beispielsweise durch explosionsgefährdete Bereiche geführt werden und müssen dabei nicht über normative Isolierstärken zur Vermeidung interner Kurzschlüsse verfügen.

Die gilt insbesondere für typische Anwendungsfälle der beschriebenen Architektur. In engen Installationsräumen wird oft ein Transmitter A an zugänglicher Stelle installiert, der auch als Messwertanzeige dient. Zugleich ist dieser Transmitter A mit der Zentrale (Controller) verbunden. Dieser Transmitter A kann sowohl eine als auch mehrere Kanäle Messkanäle und somit Verbindungen zum Controller erhalten. Oft wird ein weiterer Transmitter B über ein proprietäres Bussystem angeschlossen, dass auch die Energieversorgung eines abgesetzten Messkopfes enthält.

Zumeist sind diese abgesetzten Messköpfe mit Transmitter B an äußerst unzugänglichen Orten installiert und verfügen oft über keine eigene Messwertanzeige. Mit herkömmlichen Lösungen ist die Installationslänge zwischen dem eben beschriebenen abgesetzten Transmitter B (Messkopf) und dem Transmitter A, der die Verbindung zur Zentrale unterhält, äußerst begrenzt. Das ist vor allem der Fall, wenn Transmitter nach der Zündschutzart Eigensicherheit 60079-11 zugelassen ist. Diese Norm erzwingt starke Limitierung von Leistung.

Herkömmlich wird dies dadurch erreicht, in Transmitter A oder in Transmitter B (oder in beiden), Widerstände zu integrieren, um somit den Umladestrom zu reduzieren. Dieses führt aber zu erheblichen Verlusten und einer deutlich reduzierten Energieeffizienz sowie reduziertem Funktionsumfang.

Die hierin beschriebenen Ausführungsbeispiele erweitern die mögliche Installationslänge der Leitung für die Verbindung zwischen Transmittern A und B deutlich. Zugleich ermöglichen diese Ausführungsbeispiele ebenfalls einen abgesetzten Messkopf mit weiteren Funktionen, sowie einem Display auszustatten. Das ist ein wesentlicher Vorteil beim Service und Installation und Inbetriebnahme des Transmitters. Sie ermöglicht auch das Signale und Versorgung in einem Kabel geführt werden können. Das ist ein erheblicher Vorteil, da sicherheitstechnische Installationsvorschriften in Bezug auf Trennung und Separation entfallen. Sicherungen, die herkömmlicherweise zur Begrenzung der Leistung im Transmitter verbaut sind, werden zudem vor unabsichtlichem Auslösen geschützt.

Der integrierte zusätzliche elektronische Schutz und somit die Begrenzung der Umlade- und Fehlerströme zwischen Transmitter A und B stellt auch deswegen einen erheblichen Vorteil dar, da ein Käufer des Systems den Anschluss des abgesetzten Messkopfes selbst vornehmen kann da die Funkenbildung in jedem Fall ausgeschlossen ist.

Für eine Anwendung mit oder in einem Pumpenmodul ergeben sich ebenfalls spezifische Vorteile. Herkömmlich werden aufgrund der stark normativen Randbedingungen Pumpen für explosionsgeschützte Bereiche (auf Grund ihrer hohen Leistung) oft innerhalb einer druckfesten Kapselung (IEC 60079-1) verbaut. Bei dieser Zündschutzart ist ein Service ohne Freimessen und Abschaltung von ganzen Anlagen nicht möglich, da zu Wartungszwecken die druckfeste Kapselung geöffnet werden muss. Daher kann bei jeder Berührung von Elektronik es zum sofortigen Zünden einer explosiven Atmosphäre führen. In allen derzeit existierenden Anwendungen mit Pumpen muss bei einer Wartung aus Gründen des Explosionsschutzes der Teil einer Anlage abgeschaltet werden, was zu sehr hohen Kosten bzw. zu Ausfall der Produktion führen kann.

Durch die hierin beschriebenen Ausführungsbeispiele wäre es beispielsweise möglich, ein hotplug-fähiges Pumpenmodul mit einer Zündschutzart zu ermöglichen, dass den Tausch während des Betriebes ermöglicht. Auch eine kostengünstige und einfache Gehäusekonstruktion für das Endprodukt könnte dadurch ermöglicht werden.

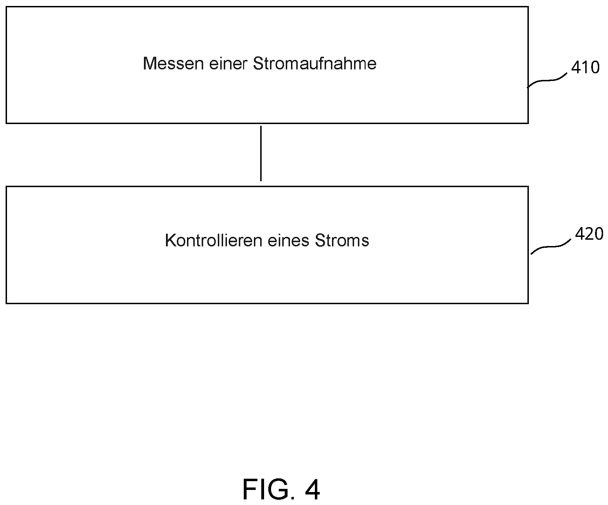

Fig. 4 zeigt ein Flussdiagramm eines Ausführungsbeispiels eines Verfahrens zur Energieversorgung eines Lastmoduls, wie es in den vorhergehenden Abschnitten, beispielsweise bezugnehmend auf Fig. 1, beschrieben wurde.

Das Verfahren umfasst ein Messen 410 einer Stromaufnahme über einem Eingangsanschluss, der an eine Energieversorgung gekoppelt ist.

Das Verfahren umfasst ferner ein Kontrollieren 420 eines Stroms über einen Ausgangsanschluss basierend auf der Stromaufnahme über den Eingangsanschluss.

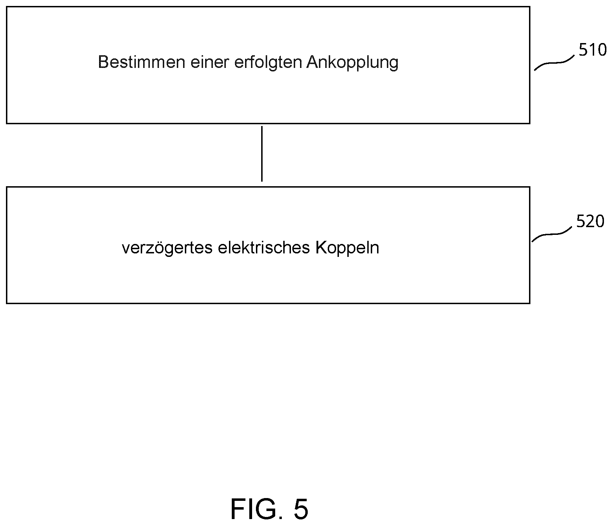

Fig. 5 zeigt ein Flussdiagramm eines Ausführungsbeispiels eines Verfahrens zur Ansteuerung einer Last, wie es in den vorhergehenden Abschnitten, beispielsweise bezugnehmend auf die Fig. 2 und 3, beschrieben wurde.

Das Verfahren umfasst ein Bestimmen 510 einer erfolgten Ankopplung eines Eingangsanschlusses an eine Energieversorgung.

Das Verfahren umfasst ferner ein verzögertes elektrisches Koppeln 520 des Eingangsanschlusses mit einem Ausgangsanschluss zur Ankopplung an den Verbraucher nach dem Messen der erfolgten Ankoppelung an die Energieversorgung.

Die Aspekte und Merkmale, die zusammen mit einem oder mehreren der vorher detaillierten Beispiele und Figuren beschrieben sind, können auch mit einem oder mehreren der anderen Beispiele kombiniert werden, um ein gleiches Merkmal des anderen Beispiels zu ersetzen oder um das Merkmal in das andere Beispiel zusätzlich einzuführen.

Beispiele können weiterhin ein Computerprogramm mit einem Programmcode zum Ausführen eines oder mehrerer der obigen Verfahren sein oder sich darauf beziehen, wenn das Computerprogramm auf einem Computer oder Prozessor ausgeführt wird. Schritte, Operationen oder Prozesse von verschiedenen, oben beschriebenen Verfahren können durch programmierte Computer oder Prozessoren ausgeführt werden. Beispiele können auch Programmspeichervorrichtungen, z. B. Digitaldatenspeichermedien, abdecken, die maschinen-, prozessor- oder computerlesbar sind und maschinenausführbare, prozessorausführbare oder computerausführbare Programme von Anweisungen codieren. Die Anweisungen führen einige oder alle der Schritte der oben beschriebenen Verfahren aus oder verursachen deren Ausführung. Die Programmspeichervorrichtungen können z. B. Digitalspeicher, magnetische Speichermedien wie beispielsweise Magnetplatten und Magnetbänder, Festplattenlaufwerke oder optisch lesbare Digitaldatenspeichermedien umfassen oder sein. Weitere Beispiele können auch Computer, Prozessoren oder Steuereinheiten, die zum Ausführen der Schritte der oben beschriebenen Verfahren programmiert sind, oder (feld-) programmierbare Logik-Arrays ((F)PLAs = (Field) Programmable Logic Arrays) oder (feld-)programmierbare Gate-Arrays ((F)PGA = (Field) Programmable Gate Arrays), die zum Ausführen der Schritte der oben beschriebenen Verfahren programmiert sind, abdecken.

Durch die Beschreibung und Zeichnungen werden nur die Grundsätze der Offenbarung dargestellt. Weiterhin sollen alle hier aufgeführten Beispiele grundsätzlich ausdrücklich nur illustrativen Zwecken dienen, um den Leser beim Verständnis der Grundsätze der Offenbarung und der durch den (die) Erfinder beigetragenen Konzepte zur Weiterentwicklung der Technik zu unterstützen. Alle hiesigen Aussagen über Grundsätze, Aspekte und Beispiele der Offenbarung sowie konkrete Beispiele derselben umfassen deren Entsprechungen.

Ein als "Mittel zum..." Ausführen einer bestimmten Funktion bezeichneter Funktionsblock kann sich auf eine Schaltung beziehen, die ausgebildet ist zum Ausführen einer bestimmten Funktion. Somit kann ein "Mittel für etwas" als ein "Mittel ausgebildet für oder geeignet für etwas" implementiert sein, z. B. ein Bauelement oder eine Schaltung ausgebildet für oder geeignet für die jeweilige Aufgabe.

Funktionen verschiedener in den Figuren gezeigter Elemente einschließlich jeder als "Mittel", "Mittel zum Bereitstellen eines Signals", "Mittel zum Erzeugen eines Signals", etc. bezeichneter Funktionsblöcke kann in Form dedizierter Hardware, z. B "eines Signalanbieters", "einer Signalverarbeitungseinheit", "eines Prozessors", "einer Steuerung" etc. sowie als Hardware fähig zum Ausführen von Software in Verbindung mit zugehöriger Software implementiert sein. Bei Bereitstellung durch einen Prozessor können die Funktionen durch einen einzelnen dedizierten Prozessor, durch einen einzelnen gemeinschaftlich verwendeten Prozessor oder durch eine Mehrzahl von individuellen Prozessoren bereitgestellt sein, von denen einige oder von denen alle gemeinschaftlich verwendet werden können. Allerdings ist der Begriff "Prozessor" oder "Steuerung" bei Weitem nicht auf ausschließlich zur Ausführung von Software fähige Hardware begrenzt, sondern kann Digitalsignalprozessor-Hardware (DSP-Hardware; DSP = Digital Signal Processor), Netzprozessor, anwendungsspezifische integrierte Schaltung (ASIC = Application Specific Integrated Circuit), feldprogrammierbare Logikanordnung (FPGA = Field Programmable Gate Array), Nurlesespeicher (ROM = Read Only Memory) zum Speichern von Software, Direktzugriffsspeicher (RAM = Random Access Memory) und nichtflüchtige Speichervorrichtung (storage) umfassen. Sonstige Hardware, herkömmliche und/oder kundenspezifische, kann auch eingeschlossen sein.

Ein Blockdiagramm kann zum Beispiel ein grobes Schaltdiagramm darstellen, welches die Grundsätze der Offenbarung implementiert. Auf ähnliche Weise können ein Flussdiagramm, ein Ablaufdiagramm, ein Zustandsübergangsdiagramm, ein Pseudocode und dergleichen verschiedene Prozesse, Operationen oder Schritte repräsentieren, die zum Beispiel im Wesentlichen in computerlesbarem Medium dargestellt und so durch einen Computer oder Prozessor ausgeführt werden, ungeachtet dessen, ob ein solcher Computer oder Prozessor explizit gezeigt ist. In der Beschreibung oder in den Patentansprüchen offenbarte Verfahren können durch ein Bauelement implementiert werden, das ein Mittel zum Ausführen eines jeden der jeweiligen Schritte dieser Verfahren aufweist.

Es versteht sich, dass die Offenbarung mehrerer, in der Beschreibung oder den Ansprüchen offenbarter Schritte, Prozesse, Operationen oder Funktionen nicht als in der bestimmten Reihenfolge befindlich ausgelegt werden soll, sofern dies nicht explizit oder implizit anderweitig, z. B. aus technischen Gründen, angegeben ist. Daher werden diese durch die Offenbarung von mehreren Schritten oder Funktionen nicht auf eine bestimmte Reihenfolge begrenzt, es sei denn, dass diese Schritte oder Funktionen aus technischen Gründen nicht austauschbar sind. Ferner kann bei einigen Beispielen ein einzelner Schritt, Funktion, Prozess oder Operation mehrere Teilschritte, -funktionen, -prozesse oder -operationen einschließen und/oder in dieselben aufgebrochen werden. Solche Teilschritte können eingeschlossen sein und Teil der Offenbarung dieses Einzelschritts sein, sofern sie nicht explizit ausgeschlossen sind.

Weiterhin sind die folgenden Ansprüche hiermit in die detaillierte Beschreibung aufgenommen, wo jeder Anspruch als getrenntes Beispiel für sich stehen kann. Während jeder Anspruch als getrenntes Beispiel für sich stehen kann, ist zu beachten, dass - obwohl ein abhängiger Anspruch sich in den Ansprüchen auf eine bestimmte Kombination mit einem oder mehreren anderen Ansprüchen beziehen kann - andere Beispiele auch eine Kombination des abhängigen Anspruchs mit dem Gegenstand jedes anderen abhängigen oder unabhängigen Anspruchs umfassen können. Solche Kombinationen werden hier explizit vorgeschlagen, sofern nicht angegeben ist, dass eine bestimmte Kombination nicht beabsichtigt ist. Ferner sollen auch Merkmale eines Anspruchs für jeden anderen unabhängigen Anspruch eingeschlossen sein, selbst wenn dieser Anspruch nicht direkt abhängig von dem unabhängigen Anspruch gemacht ist.

### Bezugszeichenliste

- 100: Versorgungsvorrichtung
- 110: Eingangsanschluss
- 120: Ausgangsanschluss
- 130: Messeinrichtung
- 140: Steuereinrichtung
- 150: Kontrolleinrichtung
- 200: Lastmodul
- 210: Eingangsanschluss (Lastmodul)
- 220: Ausgangsanschluss (Lastmodul)
- 230: Schalteinrichtung
- 240: Kontrolleinrichtung (Lastmodul)
- 250: Kondensator
- 260: Elektrischer Widerstand
- 410: Messen
- 420: Kontrollieren
- 510: Bestimmen einer Ankopplung
- 520: Verzögertes elektrisches Koppeln

## Patentansprüche

1. Ein Lastmodul (200) zur Versorgung einer Last, mit
- einem Eingangsanschluss (210) zur Ankopplung an eine Energieversorgung;
- einem Ausgangsanschluss (220) zur Ankopplung an die Last;
- einer Schalteinrichtung (230) zur variablen elektrischen Kopplung des Eingangsanschlusses (210) mit dem Ausgangsanschluss (220);
- einer Kontrolleinrichtung (240), die mit Schalteinrichtung (230) gekoppelt ist, und die ausgebildet ist, um nach einer Ankopplung des Lastmoduls (200) an eine Energieversorgung über die Schalteinrichtung (230) eine elektrische Kopplung des Eingangsanschlusses (210) mit dem Ausgangsanschluss (220) zu kontrollieren,
wobei die Kontrolleinrichtung (240) ausgebildet ist, um einen Stromfluss mittels eines geschalteten Transistors nur in einer Richtung zu ermöglichen.

2. Das Lastmodul (200) gemäß Anspruch 1,
wobei die Kontrolleinrichtung (240) ausgebildet ist, um nach der erfolgten Ankopplung an die Energieversorgung über die Schalteinrichtung (230) eine elektrische Kopplung des Eingangsanschlusses (210) mit dem Ausgangsanschluss (220) zu verzögern.

3. Das Lastmodul (200) gemäß Anspruch 2,
wobei die Kontrolleinrichtung (240) ausgebildet ist, um die verzögerte elektrische Kopplung des Eingangsanschlusses (210) mit dem Ausgangsanschluss (220) basierend auf einem Ladezustand eines Kondensators (250) durchzuführen,
wobei der Kondensator (250) optional nach Ankopplung an die Energieversorgung über einen begrenzten Strom geladen wird.

4. Das Lastmodul (200) gemäß Anspruch 3,
wobei die Schalteinrichtung (230) mit einem elektrischen Widerstand (260) überbrückt ist, um den Kondensator (250) zu laden.

5. Das Lastmodul (200) gemäß einem der Ansprüche 3 bis 4,
wobei die Kontrolleinrichtung (240) ausgebildet ist, um die Schalteinrichtung (230) hochohmig zu schalten, wenn die Ankopplung an die Energieversorgung erfolgt, und um die Schalteinrichtung (230) niederohmig zu schalten, wenn die Kondensatorspannung einen vordefinierten Schwellwert überschreitet.

6. Das Lastmodul (200) gemäß einem der Ansprüche 3 bis 5,
ferner umfassend die Last, die mit dem Ausgangsanschluss (220) gekoppelt ist,
wobei die Last ein Pumpenmodul oder einen Messkopf für Gase umfasst,
wobei das Lastmodul (200) optional druckfest gekapselt und/oder zum Einsatz in einer explosiven Atmosphäre ausgebildet ist.

7. Ein Verfahren zur Energieversorgung eines Lastmoduls (200), mit den Schritten
- Messen (410) einer Stromaufnahme über einem Eingangsanschluss, der an eine Energieversorgung gekoppelt ist;
- Kontrollieren (420) eines Stroms über einen Ausgangsanschluss basierend auf der Stromaufnahme über den Eingangsanschluss; und optional
- Bestimmen (510) einer erfolgten Ankopplung eines Eingangsanschlusses an eine Energieversorgung; und
- verzögertes elektrisches Koppeln (520) des Eingangsanschlusses mit einem Ausgangsanschluss zur Ankopplung an den Verbraucher nach dem Messen der erfolgten Ankoppelung an die Energieversorgung.
